(19) Europäisches Patentamt
European Patent Office
Office européen des brevets

(11) Veröffentlichungsnummer: **0 106 138**
**B1**

(12) # EUROPÄISCHE PATENTSCHRIFT

(45) Veröffentlichungstag der Patentschrift: **03.07.85**

(51) Int. Cl.⁴: **C 07 C 118/02**

(21) Anmeldenummer: **83108925.5**

(22) Anmeldetag: **09.09.83**

(54) **Verfahren zur kontinuierlichen Heissphosgenierung von Aminen.**

(30) Priorität: **09.10.82 DE 3237541**
**02.07.83 DE 3323882**

(43) Veröffentlichungstag der Anmeldung: **25.04.84 Patentblatt 84/17**

(45) Bekanntmachung des Hinweises auf die Patenterteilung: **03.07.85 Patentblatt 85/27**

(84) Benannte Vertragsstaaten: **AT BE CH DE FR GB IT LI LU NL SE**

(56) Entgegenhaltungen: **FR - A - 2 503 146**

(73) Patentinhaber: **CHEMISCHE WERKE HÜLS AG, - RSP Patente / PB 15 - Postfach 13 20, D-4370 Marl 1 (DE)**

(72) Erfinder: **Disteldorf, Josef, Dr., Am Sengenhoff 2a, D-4690 Herne 1 (DE)**
Erfinder: **Hübel, Werner, Dr., Birnenbruchstrasse 34, D-4690 Herne 1 (DE)**
Erfinder: **Reiffer, Johannes, Dr., Sonnenstrasse 10, D-4300 Essen 15 (DE)**

**EP 0 106 138 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

## Beschreibung

Bekanntlich wird der überwiegende Teil organischer Isocyanate durch Phosgenierung primärer Amine, ihrer Hydrochloride oder ihrer carbonamidsauren Salze hergestellt (vgl. Ullmanns Encyklopädie der technischen Chemie, 4. Auflage, Band 13, S. 350 ff.).

Technisch am bedeutsamsten ist die sogenannte Basenphosgenierung. In der Kälte reagiert das Amin, das in einem inerten Lösemittel vorliegt, mit überschüssiger Phosgenlösung (Kaltphosgenierung):

$$2RNH_2 + COCl_2 \rightarrow R-NH-CO-Cl + R-\overset{\oplus}{N}H_3Cl^{\ominus}$$

Im zweiten Schritt, der sogenannten Heissphosgenierung, spaltet das Carbamoylchlorid oberhalb von 80°C unter Bildung des Isocyanats Chlorwasserstoff ab. Oberhalb dieser Temperatur reagiert auch das Hydrochlorid zum Isocyanat:

$$R-NH-CO-Cl + R-\overset{\oplus}{N}H_3\overset{\ominus}{Cl} + COCl_2 \rightarrow 2RNCO + 4HCl$$

Konkurrenzreaktion zur Isocyanatbildung ist die Harnstoffbildung aus Amin und bereits gebildetem Carbamoylchlorid oder Isocyanat.

$$R-NHCOCl + R-NH_2 \rightarrow R-NH-CO-NH-R + HCl$$

$$R-NCO + R-NH_2 \rightarrow R-NH-CO-NH-R$$

Je nach Amin setzen sich die Harnstoffe nur sehr schwer oder praktisch gar nicht zum Isocyanat um. Das gilt besonders für aliphatische Amine und ganz besonders für Polyamine. Deshalb muss die Verfahrensführung darauf ausgerichtet sein, die Harnstoffbildung zu verhindern.

Die Vorphosgenierung wird vorzugsweise mit grossem Phosgenüberschuss bei möglichst tiefer Temperatur durchgeführt. In der Praxis legt man daher relativ konzentrierte Phosgenlösung im Überschuss vor und gibt das Amin in einem organischen Lösemittel zu.

Entscheidend ist, dass das Amin möglichst schnell in der Phosgenlösung verteilt wird. In einer Reihe von Patentschriften werden spezielle Mischvorrichtungen beschrieben. So wird hervorgehoben, dass es zur Verhinderung der Nebenreaktion des Amins mit bereits gebildetem Isocyanat darauf ankommt, «dass jedes einzelne der in das Verfahren eingeschickten Aminmoleküle augenblicklich von Phosgen umschlossen werden müsste, bevor es mit irgendwelchen gebildeten Isocyanatmolekülen in Berührung gelangt» (DE-PS 1792660, Spalte 3, Zeilen 15 bis 19). Dies ist praktisch nicht erreichbar. Die wirksamsten Mischaggregate sind Mischdüsen. Bei diesen kommt es jedoch zu Verstopfungen und Ablagerungen von Feststoffen, die den Mischvorgang stören.

Es ist ein erheblicher Aufwand erforderlich, um die bei der stark exothermen Reaktion gebildete Reaktionswärme auf einem niedrigen Temperaturniveau abzuführen. Praktisch arbeitet man meist kontinuierlich adiabatisch, also bei relativ hoher Temperatur (vgl. DE-PS 1165587). Die Bereitstellung einer hochkonzentrierten Phosgenlösung ist sicherheitstechnisch bedenklich.

Trotz der geschilderten Nachteile werden die meisten aromatischen Amine basenphosgeniert.

Aliphatische Amine, die stärker basisch sind, lassen sich noch schwieriger direkt als Base phosgenieren. Die Harnstoffbildung kann man leichter verhindern, wenn statt des freien Amins sein carbamidsaures Salz zur Vorphosgenierung eingesetzt wird.

Im sogenannten Carbamat-Verfahren bereitet man zunächst durch Sättigen einer Aminlösung mit Kohlendioxid das carbamidsaure Salz,

$$2RNH_2 + CO_2 \rightarrow R-\overset{\oplus}{N}H_3\overset{\ominus}{O}OC-NH-R,$$

das schwer löslich ist und im Lösemittel suspendiert vorliegt. Bei der anschliessenden Kaltphosgenierung bildet sich unter Abspalten von $CO_2$ das gleiche Carbamoylchlorid-Hydrochlorid-Gemisch wie in der Basenphosgenierung, das dann auch ebenso heissphosgeniert wird:

$$R-NH-COO^{\ominus}H_3\overset{\oplus}{N}-R + COCl_2 \rightarrow R-NH-COCl + R-\overset{\oplus}{N}H_3\overset{\ominus}{Cl} + CO_2$$

$$R-NH-CO-Cl + R\overset{\oplus}{N}H_3Cl^{\ominus} + COCl_2 \rightarrow 2R-NCO + 4HCl$$

Das Carbamat-Verfahren erlaubt zwar einen geringeren Aufwand beim Mischen des Amins bzw. dessen Carbamats mit Phosgen; nachhaltig ist jedoch, dass eine dritte Reaktionsstufe erforderlich ist und zusätzlich $CO_2$ benötigt wird, das sich nur unter erheblichem Aufwand aus dem Abgas zurückgewinnen lässt.

Am sichersten lässt sich die Harnstoffbildung verhindern, wenn man der sogenannten Hydrochlorid-Methode folgt und die Hydrochloride phosgeniert:

$$R-\overset{\oplus}{N}H_3Cl^{\ominus} + COCl_2 \rightarrow RNCO + 3HCl$$

Die stabilen Hydrochloride reagieren nicht in der Kälte und selbst in der Hitze ist ihre Reaktionsgeschwindigkeit vergleichsweise gering. Lange Reaktionszeiten oder zur Verkürzung der Reaktionsdauer angewandte höhere Temperaturen führen aber dazu, dass Nebenreaktionen, wie Chlorsubstitution und Isocyanatpolymerisation, ein unerwünschtes Ausmass erreichen. Eine kontinuierliche Durchführung dieser Reaktion ist daher erschwert.

In der DE-OS 1568844 wird die typische Verfahrensweise einer solchen Phosgenierung beschrieben. Die Suspension des Hydrochlorids eines organischen Amins wird bei erhöhter Temperatur in einer Rührkaskade mit mehreren Stufen phosgeniert, bis eine klare Reaktionslösung abfliesst.

Als Beispiel für die auftretenden Schwierigkeiten sei ferner die Phosgenierung von 1,5-Diamino-2-methylpentan angeführt. Der US-PS 3631198 ist zu entnehmen, dass dieses Diamin unter Bedingungen, bei denen das analoge Hexamethylendiisocyanat in 95%iger Ausbeute

erhalten wird, lediglich in einer Ausbeute von 19% isoliert wird. Gemäss der genannten US-Patentschrift lässt sich zwar durch Verwendung von Diethylphtalat die Ausbeute auf 82,7% steigern, doch hat dieses Lösemittel den Nachteil, dass sein Siedepunkt nahe dem des erhaltenen Diisocyanats liegt und somit schwer abzutrennen ist. Ausserdem ist es unter den Reaktionsbedingungen nicht hinreichend beständig. Es bildet sich Phthalsäureanhydrid, das kristallin ausfällt und stört.

Ziel der vorliegenden Erfindung war es, ausgehend von der Hydrochloridmethode, den Heissphosgenierungsschritt kontinuierlich so zu gestalten, dass die Reaktionszeit verkürzt und möglichst in einer Stufe gearbeitet werden kann. Ein weiteres Ziel war es, bei milderen Reaktionsbedingungen zu reineren Produkten und besseren Ausbeuten zu gelangen.

Es wurde jetzt gefunden, dass man das Verfahren der Heissphosgenierung von in Lösemittel suspendiertem Aminhydrochlorid oder dessen Gemisch mit Carbamoylchlorid mit überschüssigem Phosgen bei einer Temperatur zwischen 80 und 200°C in kontinuierlicher Fahrweise dadurch erheblich verbessern kann, dass man mit Hilfe einer geeigneten Trennvorrichtung die Feststoffe im Reaktor zurückhält und das während der Reaktion gebildete, im Lösemittel gelöste Isocyanat laufend aus dem Reaktor entfernt.

In kontinuierlicher Fahrweise stellt sich im Reaktor eine bestimmte Feststoffkonzentration ein, die sich durch Erhöhung bzw. Verringerung von Zu- und Abgabe verändern lässt. Die Zugabe besteht aus Aminhydrochlorid oder dessen Gemisch mit Carbamoylchlorid in einem geeigneten Lösemittel. Die Abgabe enthält das mit Hilfe der erwähnten Trennvorrichtung abgeleitete Isocyanat, das im gleichen Lösemittel gelöst vorliegt. Da sich mit Erhöhung der Feststoffkonzentration der Umsatz erhöht, wird eine möglichst hohe Konzentration angestrebt, die jedoch dadurch begrenzt ist, dass eine fliessbare Suspension erhalten bleiben muss. Je nach eingesetztem Amin fällt die optimale Feststoffkonzentration unterschiedlich aus. Während beispielsweise das Optimum beim 1,5-Diamino-2-methylpentan etwa bei 20% liegt, sind beim Isophorondiamin nur Konzentrationen von etwa 1% möglich, da sonst das Hydrochlorid verklumpen würde.

Weitere Gegenstände der Erfindung gehen aus den Ansprüchen 2 bis 7 hervor.

Das erfindungsgemässe Verfahren gestaltet sich besonders günstig bei Anwendung der Hydrochloridmethode. Die Reaktionszeiten können erheblich verkürzt werden.

Wegen der schonenden Reaktionsbedingungen, d.h. niedrige Reaktionstemperatur und kurze Reaktionszeit, lassen sich die Isocyanate in fast quantitativer Ausbeute und sehr hoher Reinheit gewinnen.

Im des 1,5-Diamino-2-methylpentans wird auf diese Weise erst eine wirtschaftliche Herstellung des Diisocyanats ermöglicht. Es gelingt, die mittlere Verweilzeit um den Faktor 4 zu senken. Bei anderen aliphatischen Diaminen erhält man mit der Hydrochloridmethode Umsätze, die sonst nur nach vorausgegangener Vorphosgenierung erzielbar sind.

Beispiel 4 belegt, dass das erfindungsgemässe Verfahren auch mit Vorteil eingesetzt werden kann, wenn eine Vorphosgenierung bereits stattgefunden hat.

Die Abtrennung der isocyanathaltigen Reaktionslösung kann auf unterschiedliche Weise erfolgen. Beispielsweise kann ein Teil der Suspension aus dem Reaktor abgezweigt und der Feststoff abgetrennt und in den Reaktor zurückgeführt werden. Die Trennung kann aber auch im Reaktor selbst bzw. vor dem Produktausgang vorgenommen werden, wobei der Feststoff ständig im Reaktor verbleibt. Die Trennung kann beispielsweise durch Dekantieren, Zentrifugieren oder mit Hilfe eines Hydrozyklons erfolgen. Vorzugsweise wird die Reaktionslösung mit Hilfe eines Filters abgetrennt.

Das erfindungsgemässe Verfahren lässt sich anwenden zur Phosgenierung primärer aliphatischer, cycloaliphatischer, araliphatischer und aromatischer Mono- und Polyamine mit 5 bis 18 C-Atomen oder deren technischen Gemischen. Beispielhaft seien genannt 1,3-Bis-(aminomethyl)-benzol, Anilin, Toluylendiamin-1,3 und -1,4, 4,4'-Diaminodiphenylmethan, Octadecylamin, 1,5,9-Triaminoundecan, Cyclopentylamin. Bevorzugt geeignet sind aliphatische und cycloaliphatische Diamine, wie 1,6-Diaminohexan, 1,5-Diamino-2-methylpentan, 1,6-Diamino-2,2,4- oder -2,4,4-trimethylhexan, 1,9-Diamino-5-methylnonan sowie 1-Amino-3-aminomethyl-3,5,5-trimethylcyclohexan.

Als Lösemittel eignen sich aliphatische und aromatische Verbindungen, die unter den Reaktionsbedingungen inert sind. Diese enthalten 6 bis 15 C-Atome und tragen als Substituenten gegebenenfalls Alkylreste und Chloratome. Beispielhaft seien genannt Toluol, Xylol, Tetralin und Decalin. Bevorzugt verwendet man Chlorbenzol mit einem Siedepunkt von 130°C oder o-Dichlorbenzol mit einem Siedepunkt von 170°C, wenn die Reaktion bei höherer Temperatur erfolgen soll. Die der Heissphosgenierung zugeführte Suspension enthält pro kg umzusetzendem Amin 3 bis 30 l, vorzugsweise 10 bis 20 l, Lösemittel.

Die meisten Hydrochloride und Carbamoylchloride insbesondere aliphatischer und cycloaliphatischer Diamine sind in den aufgeführten Lösemittel so wenig löslich, dass sie mit Hilfe der Trennvorrichtung quantitativ zurückgehalten werden. Sind die Aminsalze hingegen in nennenswertem Umfang im Lösemittel löslich, so können sie zusammen mit dem entstehenden Isocyanat den Reaktor verlassen. In solchen Fällen ist eine Nachreaktion erforderlich.

Auch in diesen Fällen weist das erfindungsgemässe Verfahren Vorteile auf. So lässt sich beispielsweise die mittlere Verweilzeit für die Heissphosgenierung von Toluylendiamin von mehr als 6 Stunden (Beispiel 1 der DE-OS 1568844) auf 2,5 Stunden (Beispiel 6) reduzieren.

Nach Beendigung der Phosgenierung wird die

das Isocyanat enthaltende Reaktionslösung in üblicher Weise aufgearbeitet, wie dies auch aus den Beispielen ersichtlich ist.

Die erfindungsgemäss erhältlichen Diisocyanate sind vielseitig einsetzbar, insbesondere eignen sie sich zur Herstellung von Polyurethanen, Polyharnstoffen und Polyamiden.

*Beispiel 1:*

In Reaktor I, einem Rührgefäss mit 0,5 l Volumen, werden stündlich 500 ml Chlorbenzollösung, die 45 g Isophorondiamin (1-Amino-3-aminomethyl-3,5,5-trimethylcyclohexan) enthält, eindosiert und 15 l HCl-Gas eingeleitet. Die Hydrochloridsuspension fliesst kontinuierlich in Reaktor II, dem Phosgenierreaktor mit 2 l Volumen, über. Bei 125°C werden stündlich 50 l Phosgen durchgeleitet. Am Bodenabzug entnimmt man über eine Glassinterplatte von 30 mm∅, Dichte Nr. 2, kontinuierliche so viel klare Reaktionslösung (ca. 500 ml), dass der Reaktorstand erhalten bleibt. Nach Abtreiben von Phosgen und Chlorwasserstoff durch Destillation unter Rückfluss, destilliert man das Lösungsmittel ab und isoliert das Isocyanat durch Destillation unter vermindertem Druck. Es bleibt ein Destillationsrückstand von 1,2%. Die analytische Ausbeute beträgt 98,5%.

*Beispiel 2:*

In Reaktor I, der Apparatur nach Beispiel 1, werden stündlich 300 ml Chlorbenzollösung, die 30 g Trimethylhexamethylendiamin (Isomerengemisch 1,6-Diamino-2,2,4(2,4,4)-trimethylhexan) enthält, eindosiert und 12 l HCl-Gas eingeleitet. Die Suspension fliesst kontinuierlich in Reaktor II über, in dem bei 122°C und einem Phosgendurchsatz von 50 l/h heissphosgeniert wird. Stündlich zieht man am Reaktorboden so viel klare Reaktionslösung ab (ca. 300 ml), dass der Reaktorstand erhalten bleibt. Die Aufarbeitung erfolgt wie in Beispiel 1 beschrieben. Beim Destillieren bleibt ein Rückstand von 1%. Die analytische Ausbeute beträgt 98,5% Trimethylhexamethylendiisocyanat.

*Beispiel 3:*

Wie in Beispiel 1 beschrieben, werden in Reaktor I stündlich 300 ml Chlorbenzollösung, die 22,5 g eines Isomerengemisches von 92% 1,9-Diamino-5-methylnonan und 8% 1,8-Diamino-2,4-dimethyloctan enthält, eindosiert und 9 l Chlorwasserstoffgas zugeleitet. Die überfliessende Suspension wird in Reaktor II bei 122°C und einem Phosgeneinsatz von 50 l/h heissphosgeniert. Am Reaktorboden zieht man stündlich so viel klare Reaktionslösung ab (ca. 300 ml), dass der Reaktorstand erhalten bleibt. Die Aufarbeitung erfolgt wie in Beispiel 1 beschrieben. Beim Destillieren bleiben 1,1% Rückstand; die analytische Ausbeute beträgt 98,5%.

*Beispiel 4:*

Die Phosgenieranlage besteht im wesentlichen aus drei hintereinander geschalteten Rührkesseln mit folgenden Volumina: I=2 l, II= 5 l, III= 30 l.

In Reaktor I sättigt man bei 25°C stündlich 3 l Chlorbenzollösung, die 150 g reines 1,5-Diamino-2-methylpentan enthält, mit überschüssigem $CO_2$ zur Carbamat-Suspension.

Die Carbamat-Suspension fliesst in Reaktor II über, in dem bei 0°C mit überschüssigem Phosgen der Umsatz zum Carbamylchlorid-Hydrochlorid-Gemisch erfolgt. Pro Stunde werden 200 l Phosgen (aus dem Abgas rückgewonnenes Phosgen + Frischphosgen) eingeleitet.

Reaktor III wird bei 125°C betrieben. Das zur Heissphosgenierung erforderliche Phosgen ist in der Reaktionssuspension, die aus Reaktor II überfliesst, enthalten. Entsprechend dem Zulauf werden 3 l klare Lösung pro Stunde abgezogen. Vor dem Bodenabzug des Reaktors befindet sich ein Maschensieb (Chrom-Nickel-Stahl, Maschenweite 0,03 mm, 180 $cm^2$ Filterfläche), das alle festen Anteile im Reaktor zurückhält. Im Reaktor stellt sich unter diesen Bedingungen ein Feststoffanteil von 18% ein. Zur Aufarbeitung destilliert man in üblicher Weise.

Das rohe Diisocyanat enthält laut Gaschromatogramm als Verunreinigung nur 0,1% 1(5)-Chlor-5(1)-isocyanato-2-methylpentan. Der Destillationsrückstand beträgt 0,8%, die analytische Ausbeute 99%.

*Beispiel 5:*

Die Phosgenierungsapparatur besteht aus 2 hintereinander geschalteten Rührkesseln I und II mit 2 bzw. 30 l Inhalt.

In Reaktor I sättigt man bei 30°C stündlich 3 l o-Dichlorbenzollösung, die 150 g eines technischen Diamingemisches enthält, mit HCl-Gas. Das Amingemisch hat folgende Zusammensetzung:

92,3% 1,5-Diamino-2-methylpentan
7,3% 1,4-Diamino-2-ethylbutan
0,4% 1,6-Diaminohexan

Die Hydrochlorid-Suspension fliesst laufend in Reaktor II über, in den bei 140°C 200 l Phosgen/h (rückgewonnenes Phosgen + Frischphosgen) eingeleitet werden. Wie in Beispiel 4 beschrieben, zieht man stündlich 3 l klare Reaktionslösung ab. Im Reaktor stelt sich dabei ein Feststoffanteil von 20% ein. Das rohe Diisocyanat enthält nach Gaschromatogramm 0,9% Monochlorisocyanate. Der Destillationsrückstand beträgt 3,7%, die analytische Ausbeute 95%.

*Beispiel 6:*

Wie in Beispiel 1 beschrieben, werden in Reaktor I stündlich 1000 ml Chlorbenzollösung, die 100 g eines Isomerengemisches von 65% 2,4-Diaminotoluol und 35% 2,6-Diaminotoluol enthält, eindosiert und 45 l Chlorwasserstoff zugeleitet. Die überfliessende Suspension wird in Reaktor II bei 120°C und einem Phosgeneinsatz von 70 l/h heissphosgeniert. Am Reaktorboden zieht man über eine Glassinterplatte von 60 mm ∅, Dichte Nr. 3, so viel klare Reaktionslösung ab (ca. 1000 ml/h), dass der Reaktorstand erhalten bleibt. Die klare Reaktionslösung durchläuft eine beheizte Bodenkolonne mit 5 Böden und einem «holdup» von insgesamt 500 ml. Bei 125°C werden 30 l Phosgen pro Stunde von unten nach oben gegen den Flüssigkeitsstrom geleitet. Die weitere Aufarbeitung erfolgt wie in Beispiel 1 beschrieben. Beim Destillieren bleiben 6,6% Rückstand; die analytische Ausbeute beträgt 93,2%.

## Patentansprüche

1. Verfahren zur kontinuierlichen Heissphosgenierung von in Lösemittel suspendiertem Aminhydrochlorid oder dessen Gemisch mit Carbamoylchlorid mit überschüssigem Phosgen bei einer Temperatur zwischen 80 und 200°C, vorzugsweise zwischen 100 und 180°C, dadurch gekennzeichnet, dass man mit Hilfe einer geeigneten Trennvorrichtung die Feststoffe im Reaktor zurückhält und das während der Reaktion gebildete, im Lösemittel gelöste Isocyanat laufend aus dem Reaktor entfernt.

2. Verfahren nach Anspruch 1, dadurch gekennzeichnet, dass man durch Regelung des Durchsatzes den Feststoffanteil der Suspension im Reaktor so einstellt, dass die Menge des pro Zeiteinheit gebildeten Isocyanats ein Maximum erreicht.

3. Verfahren nach den Ansprüchen 1 und 2, dadurch gekennzeichnet, dass man die Trennung mit Hilfe eines Filters vornimmt.

4. Verfahren nach den Ansprüchen 1 bis 3, dadurch gekennzeichnet, dass man in den Reaktor kontinuierlich eine Suspension einspeist, deren Feststoffanteil zwischen 3 und 30%, vorzugsweise zwischen 10 und 20%, liegt.

5. Verfahren nach den Ansprüchen 1 bis 4, dadurch gekennzeichnet, dass man primäre aliphatische, cycloaliphatische, araliphatische und aromatische Mono- und Polyamine mit 5 bis 18 C-Atomen phosgeniert.

6. Verfahren nach Anspruch 5, dadurch gekennzeichnet, dass man primäre aliphatische Diamine mit 5 bis 12 C-Atomen phosgeniert.

7. Verfahren nach Anspruch 6, dadurch gekennzeichnet, dass man 1,5-Diamino-2-methylpentan phosgeniert.

## Claims

1. A process for the continuous hot phosgenation of an amine hydrochloride or an amine hydrochloride/carbamoyl chloride mixture with excess phosgene at a temperature of 80 to 200°C, preferably 100 to 180°C, the hydrochloride or mixture being suspended in a solvent, characterised in that the solid material is retained in the reactor with the aid of an appropriate separation device and the isocyanate that is formed during the reaction and is dissolved in the solvent is continuously removed from the reactor.

2. A process according to claim 1, characterised in that the solids content of the suspension in the reactor is maintained, by regulation of the throughput, at a level such that the amount of isocyanate formed per unit time is maximised.

3. A process according to claim 1 or 2, characterised in that the separation is achieved with the aid of a filter.

4. A process according to any of claims 1 to 3, characterised in that a suspension whose solids content is from 3 to 30%, preferably from 10 to 20%, is continuously fed into the reactor.

5. A process according to any of claims 1 to 4, characterised in that a primary, aliphatic, cycloaliphatic, araliphatic or aromatic mono- or polyamine of 5 to 18 carbon atoms is phosgenated.

6. A process according to claim 5, characterised in that a primary aliphatic diamine of 5 to 12 carbon atoms is phosgenated.

7. A process according to claim 6, characterised in that 1,5-diamino-2-methylpentane is phosgenated.

## Revendications

1. Procédé de phosgénation continue à chaud de chlorhydrate d'amine en suspension dans du solvant, ou de son mélange avec du chlorure de carbamoyle, au moyen de phosgène en excès, à une température comprise entre 80 et 200°C, de préférence entre 100 et 180°C, caractérisé par le fait qu'à l'aide d'un dispositif séparateur approprié on retient les solides dans le réacteur et que l'on retire continuellement du réacteur l'isocyanate formé pendant la réaction et dissous dans le solvant.

2. Procédé selon la revendication 1, caractérisé par le fait qu'en réglant le débit, on règle la proportion de solides de la suspension dans le réacteur, de façon telle que la quantité de l'isocyanate formé par unité de temps atteigne un maximum.

3. Procédé selon les revendications 1 et 2, caractérisé par le fait que l'on effectue la séparation à l'aide d'un filtre.

4. Procédé selon les revendications 1 à 3, caractérisé par le fait que l'on introduit de façon continue dans le réacteur une suspension dont la proportion de solides se situe entre 3 et 30%, de préférence entre 10 et 20%.

5. Procédé selon les revendications 1 à 4, caractérisé par le fait que l'on phosgène des monoamines et des polyamines primaires aliphatiques, cycloaliphatiques, araliphatiques et aromatiques contenant de 5 à 18 atomes de carbone.

6. Procédé selon la revendication 5, caractérisé par le fait que l'on phosgène des diamines aliphatiques primaires contenant de 5 à 12 atomes de carbone.

7. Procédé selon la revendication 6, caractérisé par le fait que l'on phosgène le 1,5-diamino-2-méthylpentane.